(19) **Europäisches Patentamt European Patent Office Office européen des brevets**

(11) **EP 4 216 812 B1**

(12) **EUROPEAN PATENT SPECIFICATION**

(45) Date of publication and mention
of the grant of the patent:
**26.11.2025 Bulletin 2025/48**

(21) Application number: **21773843.4**

(22) Date of filing: **17.09.2021**

(51) International Patent Classification (IPC):
*A61B 5/08* (2006.01)   *A61B 5/00* (2006.01)

(52) Cooperative Patent Classification (CPC):
**A61B 5/0816; A61B 5/7253; A61B 5/7257;**
A61B 2562/0219

(86) International application number:
**PCT/EP2021/075570**

(87) International publication number:
**WO 2022/063688 (31.03.2022 Gazette 2022/13)**

(54) **A PROCESSOR AND METHOD FOR DETERMINING A RESPIRATORY SIGNAL**

PROZESSOR UND VERFAHREN ZUR BESTIMMUNG EINER ATEMFREQUENZ

PROCESSEUR ET PROCÉDÉ POUR DÉTERMINER UNE FRÉQUENCE RESPIRATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2020 EP 20198259**

(43) Date of publication of application:
**02.08.2023 Bulletin 2023/31**

(73) Proprietor: **Koninklijke Philips N.V.**
**5656 AG Eindhoven (NL)**

(72) Inventors:
• **SCHIPPER, Alphonsus, Tarcisius, Jozef, Maria**
**5656 AE Eindhoven (NL)**
• **DERKX, Rene, Martinus, Maria**
**5656 AE Eindhoven (NL)**
• **VAN SLOUN, Ruud, Johannes, Gerardus**
**5656 AE Eindhoven (NL)**

(74) Representative: **Philips Intellectual Property & Standards**
**High Tech Campus 52**
**5656 AG Eindhoven (NL)**

(56) References cited:
EP-A1- 2 263 532     WO-A1-2015/018752
US-A1- 2009 062 628

• HUSSEIN ABDULRAHMAN: "Dimensionality Reduction For Dummies - Part 1: Intuition | by Hussein Abdulrahman | Towards Data Science", 18 October 2018 (2018-10-18), pages 1 - 8, XP055873589, Retrieved from the Internet <URL:https://towardsdatascience.com/ https-medium-com-abdullatif-h-dimensionality-r eduction-for-dummies-part-1-a8c9ec7b7e79> [retrieved on 20211216]
• VOEGTLIN ET AL: "Recursive principal components analysis", NEURAL NETWORKS, ELSEVIER SCIENCE PUBLISHERS, BARKING, GB, vol. 18, no. 8, 1 October 2005 (2005-10-01), pages 1051 - 1063, XP027815938, ISSN: 0893-6080, [retrieved on 20051001]
• "Engineering Statistics Handbook", 27 June 2012 (2012-06-27), XP055782473, Retrieved from the Internet <URL:https://doi.org/10.18434/ M32189>

**Description**

FIELD OF THE INVENTION

This invention relates to the generation of a respiratory signal, for example for the measurement of a respiratory rate, respiratory effort or for detecting other conditions such as sleep disordered breathing events.

BACKGROUND OF THE INVENTION

The characteristics of respiration of a subject can be of interest for detecting and managing various conditions.
For example, the respiratory rate has proven to be a good indicator of the deterioration of the condition of a patient. In combination with other vital signs, the respiratory rate plays a crucial role in early warning systems. Therefore, there is a need for continuous and reliable monitoring of a respiration signal, from which the respiratory rate can be extracted, in intensive care units of hospitals. A similar need is present in the general ward setting of hospitals and in home healthcare applications, such as in telemedicine and chronic disease management. Furthermore, the estimation of respiratory effort is important in the diagnosis of Obstructive Sleep Apnea Syndrome. The respiratory effort may be defined as the energy-consuming activity of the respiratory muscles aimed at driving respiration.
Respiration monitoring can be based on different principles: the measurement of respiratory effort, for example thorax impedance plethysmography, respiratory inductance plethysmography, accelerometers, photoplethysmography, the measurement of respiratory effect, for example sound recording, temperature sensing or carbon dioxide sensing via exhaled air via a nasal cannula, or diaphragm or parasternal muscle EMG measurement.
Some sensors are already well established to monitor respiration. In intensive care units for example, thorax impedance plethysmography is the method of choice, whereas in sleep studies respiratory inductance plethysmography, often referred to as respiration band or respiband, is also commonly used.
For ambulatory patients, such as on the general ward or in home healthcare, these sensors have limitations. A respiration band, for example, is considered to be too obtrusive and cumbersome by both medical personnel and patients. However, in many applications, ambulatory monitoring over a prolonged period is desired. A device that measures chest or rib motion is quite suitable, as it easy to wear and only requires a single point of mechanical contact with the chest. No electrical contact is required. Due to the low power consumption, the device can be quite small.
WO 2015/018752 for example discloses a method and system for obtaining a respiration signal using a chest worn device accelerometer for detecting of respiratory movements. The analysis of the movement signals involves determination of a rotation axis and/or rotation angle of that movement. A rotation model models the respiratory movement as a rotation around a single rotation axis.
It is known to use a gyroscope in addition to accelerometers, and this may be needed under certain postures, where the accelerometer does not perform well.
Respiratory rate is normally determined by either frequency analysis or time-domain analysis. Typically. some prior knowledge is assume about which directions of respiratory motion correspond to inspirations and which directions of motion correspond to expirations. However, needing this prior knowledge limits the freedom of the application. For example, both the location of attachment to the chest as well as the orientation of the sensor must be controlled in this case.
EP 2 263 532 discloses a motion determination apparatus which uses a multi-axis accelerometer to obtain acceleration signals along different spatial axes. The accelerometer signals from the different axes are combined to derive a 1 dimensional signal. The processing is based on principal component analysis and independent component analysis to find the weights of a linear transformation, which maps the 3D acceleration values onto a 1D signal space. A further example is described in US 2009/062628 A1.
There is generally a desire to reduce the level of estimation error and therefore lack of robustness in such systems, and is it always of interest to simplify sensor designs and processing requirements. The invention has these objectives.

SUMMARY OF THE INVENTION

The invention is defined by the claims.
According to examples in accordance with an aspect of the invention, there is provided a processor for deriving a respiratory signal, comprising:
an input for receiving a 3-axis acceleration signal, wherein the processor is adapted to:

> isolate a gravity vector from the 3-axis acceleration signal;
> perform a coordinate transformation into a transformed 3-axis coordinate system in which a time-averaged gravity vector is aligned with a first axis of the transformed 3-axis coordinate system;

perform analysis of the components for the remaining two axes of the transformed 3-axis coordinate system, thereby to derive a 1 dimensional respiratory signal.

The processing is used to analyze respiratory patterns in a 2D (horizontal) plane of the transformed 3-axis coordinate system, i.e. discarding the first (vertical) axis. This simplifies the processing. The transformed 3-axis coordinate system is a coordinate system of three orthogonal unit vectors. Similarly, the accelerometer generates a set of three orthogonal acceleration signals. By projecting the 3D-signal to the horizontal plane in this transformed coordinate space, additional robustness is gained. The number of dimensions is reduced prior to the processing to derive the 1D respiration signal, and this reduces computational complexity. In particular, the projection into a 2D space (when disregarding the first axis) is used before mapping the 2D acceleration values into a 1D signal. The physiological signal of interest primarily manifests itself in the 2D space. The projection thus serves as a constraint in the mapping and increases robustness, as the dimension that is discarded during the projection predominantly contains noise and no information on respiration.

The respiratory signal may be used for various purposes.

In a first set of examples, the processor of claim is further adapted to process the respiratory signal to determine a respiration rate from the 1 dimensional respiratory signal using frequency analysis.

Frequency analysis is thereby used in combination with quality-based statistical post-processing. The quality-based post-processing for example involves multiple breathing rate estimates being combined into one estimate (e.g. one per minute based on a weighted sum of 12 individual rates derived from 5 second periods). The weights may be quality numbers of the individual rates.

The processor can be used without assuming any attachment position or sensor orientation. It estimates the direction of inspirations and expirations from acceleration data captured with any orientation.

The processor may be adapted to determine the respiration rate from the 1 dimensional respiratory signal by applying a Fourier transform over a sliding time window and determining the frequency of the highest peak in the spectrum of each window, wherein the respiration rate is derived from the highest peaks for a set of successive windows.

In a second set of examples, the processor is further adapted to process the respiratory signal to determine sleep disordered breathing (SDB) events from the 1 dimensional respiratory signal.

The respiratory signal may be used, without needing to identify the actual respiration rate, to identify sleep disordered breathing events such as hypopneas, and obstructive and central apneas.

The sleep disordered breathing events may be obtained by extracting features from the 1 dimensional respiratory signal. These features may relate to amplitudes, frequencies or pattern characteristics of the signal. A machine classifier may be used to determine the events, and it may indicate a flag or probability of different sleep disordered breathing events. The SDB severity may also be derived, such as the apnea-hypopnea index (AHI).

The processor may be adapted to determine sleep disordered breathing events additionally based on analysis of a cardiac signal. For example, the inter-beat interval (IBI) or other measures may further assist in identifying sleep disordered breathing events.

In all examples, the processor may be adapted to perform pre-processing of the acceleration signal before isolating the gravity vector, wherein the pre-processing comprises sampling and low-pass filtering. This is used to extract signals of interest from the raw acceleration signal.

The processor may be adapted to isolate the gravity vector from the 3-axis acceleration signal by implementing a recursive exponential smoothing function. This provides one computationally light method for isolating the gravity vector.

The processor may perform the coordinate transformation into the transformed 3-axis coordinate system by deriving a rotation matrix which maps the isolated gravity vector to the first axis. This provides a simple computational step to align the gravity vector with one of the unit vectors of the transformed coordinate space.

The processor may be adapted to perform the analysis to derive the 1 dimensional respiratory signal using principal component analysis or using machine learning. The 1 dimensional respiratory signal is an un-calibrated estimate of the breathing effort, i.e. the instantaneous depth (and direction) of breathing.

The processor may for example be adapted to estimate the inspiratory and expiratory directions from the 1 dimensional respiratory signal. The 1 dimensional signal does not include any identification of the inspiratory and expiratory phases, but this information is needed to enable a proper analysis of the breathing characteristics of the user.

The inspiratory and expiratory directions may be estimated by determining a skewness value of the 1 dimensional respiratory signal. The skewness may for example be calculated in an iterative way.

The processor may be adapted to perform an inversion if needed, such that one type of skewness (e.g. left-skewed or right-skewed) corresponds to the inspiratory direction and another type of skewness (e.g. right-skewed or left-skewed) corresponds to the expiratory direction. This simplifies analysis of the resulting signal in that it has a known relationship with inspiratory and expiratory phases of breathing.

The invention also provides a system for deriving a respiratory signal, comprising:

an accelerometer for generating a 3-axis acceleration signal; and

the processor as defined above.

The invention also provides a method for deriving a respiratory signal, comprising:

receiving a a 3-axis acceleration signal;
isolating a gravity vector from the 3-axis acceleration signal;
performing a coordinate transformation into a transformed 3-axis coordinate system in which a time-averaged gravity vector is aligned with a first axis of the transformed 3-axis coordinate system;
performing analysis of the components for the remaining two axes of the transformed 3-axis coordinate system, thereby to derive a 1 dimensional respiratory signal.

The method is simple to implement and has good performance.
The method may comprise performing pre-processing of the acceleration signal before isolating the gravity vector, wherein the pre-processing comprises sampling and low-pass filtering.
The method may also comprise one or more of:

isolating the gravity vector from the 3-axis acceleration signal by implementing a recursive exponential smoothing function;
performing the coordinate transformation into the transformed 3-axis coordinate system by deriving a rotation matrix which maps the isolated gravity vector to the first axis;
performing the analysis to derive the 1 dimensional respiratory signal using principal component analysis or using machine learning.

An iterative principal component analysis calculation is for example low in computational cost and leverages consistency over time.
The method may comprise estimating the inspiratory and expiratory directions from the 1 dimensional respiratory signal by determining a skewness value of the 1 dimensional respiratory signal.
The method may comprise determining a respiration rate from the 1 dimensional respiratory signal using frequency analysis. The respiration rate may be derived from the 1 dimensional respiratory signal by applying a Fourier transform over a sliding time window and determining the frequency of the highest peak in the spectrum of each window, and deriving the respiration rate from the highest peaks for a set of successive windows
The method may instead comprise determining sleep disordered breathing events from the 1 dimensional respiratory signal. Sleep disordered breathing events may be determined by extracting features from the 1 dimensional respiratory signal.
Sleep disordered breathing events may be identified additionally based on analysis of a cardiac signal.
The invention may be implemented in software, and hence the invention also provides a computer program comprising computer program code which is adapted, when said computer program code is run on a computer, to implement the method defined above.
These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows the measurement of orientation with an accelerometer;
Figure 2 shows a respiratory pattern in the accelerometer coordinate system;
Figure 3 shows the same pattern as Figure 2 in an alternative coordinate system;
Figure 4 shows a horizontal projection of the 3D acceleration pattern from Figure 2 into the horizontal plane in the alternative coordinate system;
Figure 5 shows the speed profile (the bottom pane) in relation to a reference signal (the top pane);
Figure 6 shows the processing steps which are used to process the 3D acceleration data in an example of the system and method of the invention;
Figure 7 shows an estimate of the respiratory signal;
Figure 8 shows the frequency distribution of the estimate;
Figure 9 is used to show how the respiration direction is derived;

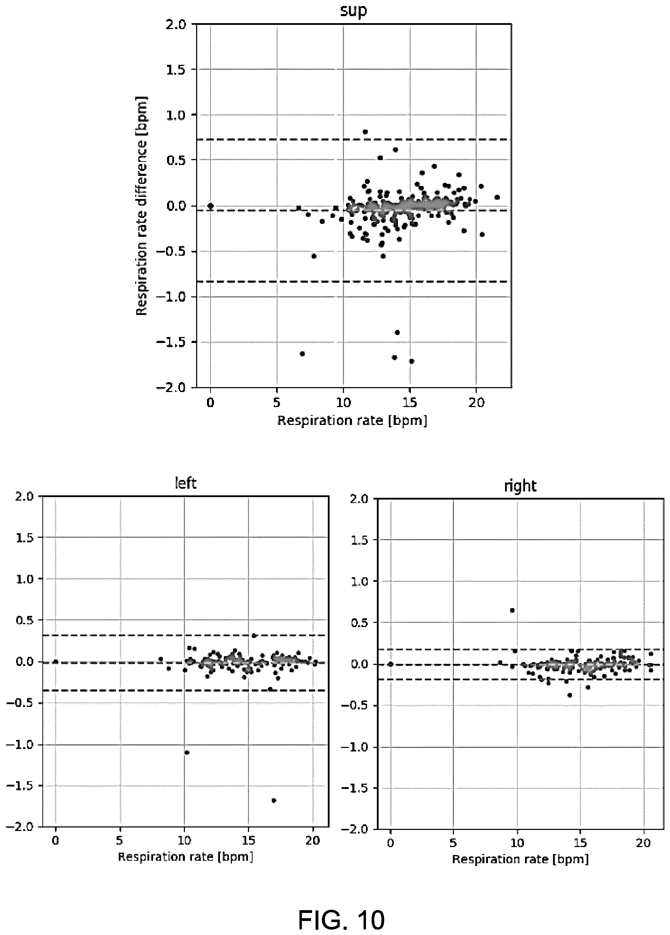

Figure 10 shows three plots to show the show the difference between the respiratory rate estimated using the processing of the invention and the respiration rate of the reference signal;

Figure 11 shows a system for detecting SDB events, from a 1D respiratory effort signal;

Figure 12 shows a possible implementation of the system of Figure 11 based on a neural network;

Figure 13 shows a set of possible signals present in the system of Figure 12; and

Figure 14 shows an extension to the example of Figure 11 in which the detected SDB events are also processed to estimate a level of SDB severity.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processor, a system using the processor and a method, for deriving a respiratory signal by processing a 3-axis acceleration signal. A gravity vector is isolated from the 3-axis acceleration signal and a coordinate transformation is performed into a transformed 3-axis coordinate system in which the isolated gravity vector is aligned with a first axis of the transformed 3-axis coordinate system. Analysis is then performed only of the components for the remaining two axes of the transformed 3-axis coordinate system, thereby to derive a 1 dimensional respiratory signal. A respiration rate may for example be obtained from the 1 dimensional respiratory signal using frequency analysis, or sleep disordered breathing events may be identified.

The system of the invention makes use of respiration measurements using an accelerometer. Respiration causes the sternum and ribs to rotate, and these movements are detected by the accelerometer.

During inspiration, the sternum and ribs move in a ventral and cranial direction. During expiration, the sternum and ribs move in the opposite direction. An attached accelerometer can measure this motion through orientation changes. The accelerometer operates in a quasi-static way and the projection of the gravity vector $\vec{g}$ on the axes is measured.

An accelerometer can be modeled as a box with a weight that is suspended inside of it. An accelerometer measures the force applied by the box to the weight. A 3D-accelerometer has three orthogonal axes $\{\vec{x}, \vec{y}, \vec{z}\}$, which form a right-handed coordinate system. An axis measures the projection of the acceleration on it.

If an accelerometer is in free fall, the box does not apply any force to the weight, and all axes measure zero acceleration.

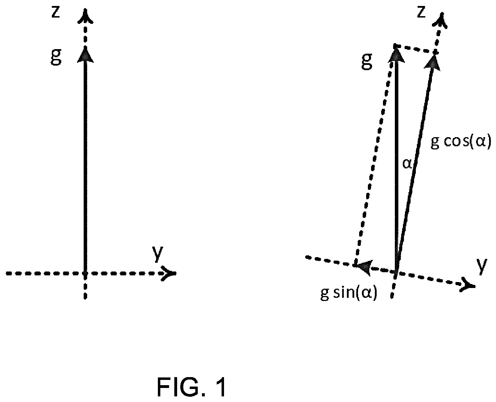

Figure 1 shows the measurement of orientation with an accelerometer. The Figure shows the axes y and z of the accelerometer. The x-axis points to the reader and is not shown. The gravity vector g is shown as g.

The left image shows the accelerometer oriented horizontally, and the z-axis is aligned with gravity. The right image shows the accelerometer tilted to the right by an angle $\alpha$. The decomposition of the gravity vector along the y- and z-axes is shown.

If an accelerometer is placed on a horizontal surface as shown in the left image, the box applies an upward force to the weight. The accelerometer measures this as an acceleration of +1 g in the upward direction. If $\alpha$ is the angle between the z-axis of the accelerometer and the upward direction as shown in the right image, then $a_z = g\ cos\ (\alpha)$. If the upward direction is in the y-z plane then $a_y = g\ sin\ (\alpha)$.

If $\alpha$ is small then $a_z \approx g$ and $a_y \approx 0$. If $\alpha$ changes by a small amount then $\Delta a_z \approx 0$ and $\Delta a_y \approx \alpha$ g. This is only true if the z-axis of the accelerometer points upward, which generally is not the case. As explained below, the invention makes use of a coordinate transformation to a new coordinate system $\{\vec{h_1}, \vec{h_2}, \vec{v}\}$. The new coordinate system forms an orthonormal, right-handed basis such that $\vec{v}$ points upward (aligns with $\vec{g}$). As a consequence, $\vec{h_1}$ and $\vec{h_2}$ lie in the horizontal plane (if $\vec{v}$ is defined/named as a vertical axis in the transformed coordinate system).

In such a case, it suffices to only measure the projection onto the horizontal plane. In other words, it suffices to use only the $\vec{h_1}$ and $\vec{h_2}$ components of the acceleration signal. This will only be valid if the accelerometer is operated in a quasi-static mode and if angles are small.

This approach can increase the robustness of the system. When deriving the respiratory signal, a model is used of which the parameters are estimated. By using this horizontal projection, the parameter space is constrained.

The parameters are the three coordinates of the projection axis. To obtain a one-dimensional respiratory waveform, the three-dimensional acceleration value is projected upon a projection axis. First, there is projection to the horizontal plane and then there is projection to an axis in that plane. The direction of the axis is such that increasing projection values correspond to inspiration.

Note that an accelerometer can only measure orientation changes if the projection of $\vec{g}$ on its axes changes. If the accelerometer is rotated around $\vec{g}$ then the projection on all of the accelerometer axes remains unchanged and nothing can be measured. A gyroscope is not susceptible to this as it can measure rotations irrespective of the orientation. A gyroscope is thus typically used for measuring orientation changes.

Respiratory patterns may be measured from the acceleration data, for example from an accelerometer placed on the chest of a subject.

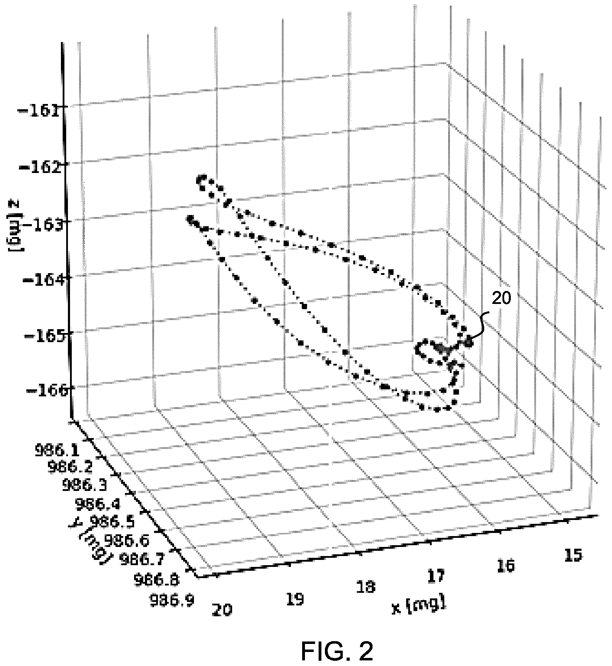

Figure 2 shows a respiratory pattern in the accelerometer coordinate system $\{\vec{x}, \vec{y}, \vec{z}\}$. The orientation of the graph is according to these axes (the z-axis is drawn vertically).

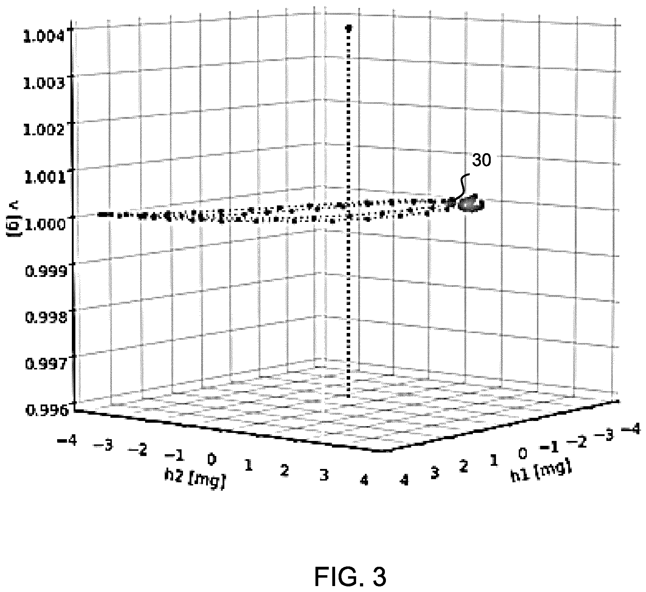

Figure 3 shows the same pattern, but in the alternative transformed coordinate system $\{\vec{h_1}, \vec{h_2}, \vec{v}\}$ explained above. In this coordinate system, $\vec{h_1}$ and $\vec{h_2}$ lie in a horizontal plane and $\vec{v}$ points upward (aligns with $\vec{g}$) and is thus the vertical line shown. It can be seen that the pattern lies in a horizontal plane, at v = 1 g.

The acceleration data for Figures 2 and 3 is sampled at 16 Hz, and the duration is 15 seconds. The data fragment spans two respiratory cycles. Each cycle forms a kind of lasso-pattern. A dotted line connects successive samples.

The dots 20, 30 indicate the start of the pattern. The axes x, y, and z are the original accelerometer axes in Figure 2, whereas Figure 3 shows the same data fragment, but then in the new coordinate system (h1, h2, v).

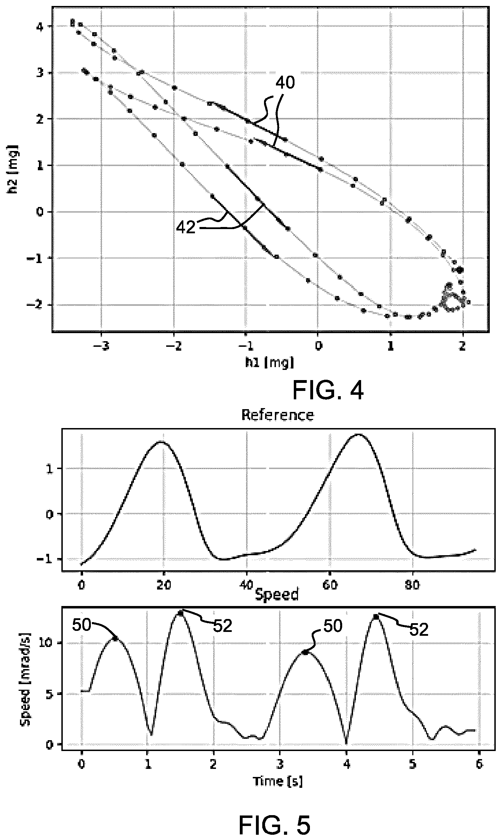

Figure 4 shows a horizontal projection of the 3D acceleration pattern from Figure 3 into the horizontal plane (h1 and h2 components), i.e. by discarding the v axis. The two respiratory cycles follow a diagonal, counter-clockwise pattern. The distance between the dots is proportional to the angular speed at which the orientation of the accelerometer changes. The further apart the dots are, the higher the speed is.

The arrows 40, 42 show the points and direction of maximum speed. The arrows 40 correspond to inspiration, and the arrows 42 correspond to expiration. Inspiration corresponds to the left and upward direction in the horizontal projection graph.

Figure 5 shows the speed profile (the bottom pane) in relation to the reference signal (the top pane).

The reference signal is an actual breathing signal as measured by other trusted sensors, and is provided for the purposes of explaining the operation of the system. It is not an available signal in the normal use of the system. The reference signal is for example obtained from a band for Respiratory Impedance Plethysmography (RIP band). Such a band is normally not used under ambulatory conditions.

The peaks in the rotational speed are at the maximum inspiratory and expiratory flow, i.e. the steepest parts of the inspiration cycle and the steepest parts of the expiration cycle in the reference signal. The peaks 50 are the inspiratory peaks and the peaks 52 are the expiratory peaks, corresponding to the vectors 40, 42.

The results presented in this application are based on a data collection study. Ten healthy subjects were used, with 80 minutes of analysis for each subject in supine, left and right postures. A chest-worn accelerometer was employed at different body positions, with voluntary breathing of the users.

In one example, the aim of the system is to obtain a respiratory signal and a respiration rate from a 3D acceleration signal.

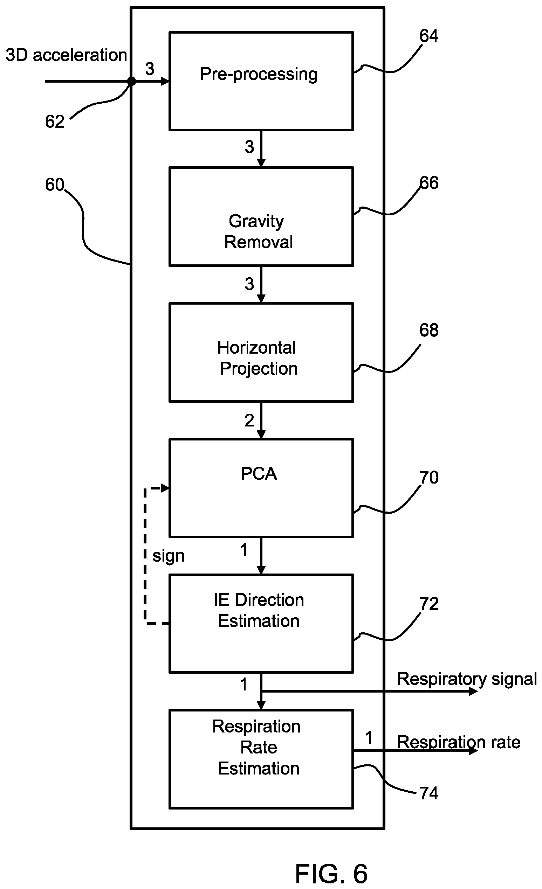

Figure 6 shows the processing steps which are used to process the 3D acceleration data.

The numbers at the input to each processing state show the dimensionality of the signals.

The processing steps are all implemented by a processor 60. The processor 60 receives as input 62 the 3 dimensional (i.e. 3-axis) acceleration signal.

In a first, pre-processing, step 64, the 3D acceleration signal is sampled, for example at 250 Hz. Respiratory patterns for example have signal components up to around 1.0 Hz, so this represents a high degree of over-sampling. Therefore, the signals are also low-pass filtered, for example with a second-order Butterworth filter with a cutoff point of 1 Hz, and are decimated to a lower sampling rate, for example to 16 Hz. The computational complexity of subsequent steps is thereby significantly reduced.

In step 66, the filtered signals are processed to perform isolation of the gravity vector gravity and removal of the gravity vector. The respiratory components in the 3D acceleration signals are relatively small compared to the gravity components. Therefore, it is advantageous first to remove the gravity components.

There are various possible methods for gravity removal.

One possible method is based on a recursive process by which an estimate is recursively adjusted until a match is found. First, gravity is estimated through exponential smoothing:

$$\overrightarrow{g_{n+1}} = \alpha \, \overrightarrow{a_n} + (1 - \alpha) \, \overrightarrow{g_n}$$

In this formula, for time step n, $\overrightarrow{g_n}$ is the gravity estimate, and $\overrightarrow{a_n}$ is the 3D acceleration. $\alpha$ is a smoothing parameter. It may for example be set such that adaptation to changes in gravity and resilience against artifacts are balanced. Typically the value of $\alpha$ is low, such as below 0.1. A value of 0.0652 in combination with a sampling rate of 16Hz is used in this analysis.

The estimated gravity is subtracted from the 3D acceleration signal, such that a 'zero-gravity' acceleration (with removal of a time-averaged gravity vector) is obtained:

$$\overrightarrow{a_n^0} = \overrightarrow{a_n} - \overrightarrow{g_n}$$

The horizontal projection mentioned above is performed in step 68. As explained above, a respiratory pattern takes place in the horizontal plane (in the transformed coordinate system).

Another possible method may operate on blocks of data, by subtracting the mean acceleration over the block.

The gravity vector is "isolated". It may be removed before the coordinate transformation, but the projection to the horizontal plane after the coordinate transformation also removes the gravity vector. Thus, removal of the gravity vector (in particular a time averaged value) may be implemented in different ways.

The respiratory pattern analysis can thus be restricted to the horizontal plane. The generation of the horizontal projection first involves performing a transformation to the new coordinate system $\{\overrightarrow{h_1}, \overrightarrow{h_2}, \overrightarrow{v}\}$ in which the axes $\overrightarrow{h_1}$ and $\overrightarrow{h_2}$ lie in the horizontal plane.

The vertical axis $\overrightarrow{v}$ can then be discarded.

The first step is to identify the components of the gravity vector $\overrightarrow{g}$ in the original accelerometer coordinate system.

This information is already obtained as part of the gravity removal step.

Using $\overrightarrow{g} = [g_x, g_y, g_z]^T$, a rotation matrix $R$ is created, such that:

$$\overrightarrow{a_h} = R\,\overrightarrow{a},$$

wherein $\overrightarrow{a}$ is the acceleration in $\{\overrightarrow{x}, \overrightarrow{y}, \overrightarrow{z}\}$ and $\overrightarrow{a_h}$ the acceleration in the transformed coordinate system $\{\overrightarrow{h_1}, \overrightarrow{h_2}, \overrightarrow{v}\}$.

The Rotation R corresponds to two successive rotations. First around the x-axis $(R_1)$ and then around the y-axis $(R_2)$:

$$R = \frac{1}{\rho} R_2 R_1,$$

with:

$$R_1 = \begin{bmatrix} \rho & 0 & 0 \\ 0 & g_z & -g_y \\ 0 & g_y & g_z \end{bmatrix} \qquad R_2 = \begin{bmatrix} \rho & 0 & g_x \\ 0 & 1 & 0 \\ -g_x & 0 & \rho \end{bmatrix} \qquad \rho = \sqrt{g_z{}^2 + g_y{}^2}$$

For the subsequent analysis, only the $\overrightarrow{h_1}$ and $\overrightarrow{h_2}$ components of $\overrightarrow{a_h}$ are used, such that the number of dimensions is reduced to two as shown in Figure 6.

Principal Component Analysis is used in step 70. The first objective is to estimate a respiratory signal $\hat{r}$ that resembles the reference signal (r) shown in Figure 5 as much as possible. The signal $\hat{r}$ is one-dimensional, while the acceleration signal is three-dimensional. Hence, the number of dimensions needs to be reduced.

Principal Component Analysis is a well-known tool for this type of data processing, and it implements Hebbian Learning. PCA is for example discussed in "Generalizations of Principal Component Analysis, Optimization Problems, and Neural Networks", J. Karhunen and J. Joutsensalo, Neural Networks, Vol. 8, No. 4, pp 549-562, 1995.

In Hebbian learning, neural paths that cause high activations are reinforced through the feedback of the activation. As only one dimension is needed, only the first principal component is required and so-called Oja's rule can be used.

Oja's rule works iteratively. Per time step, one iteration is executed. In each iteration, new a new input sample is projected onto a projection axis $\overrightarrow{w}$, such that the one-dimensional signal $\hat{r}$ can be estimated:

$$\widehat{r_n} = \overrightarrow{w_n} \cdot \overrightarrow{a_n}$$

The above formula is a dot-product of two vectors and the result $\hat{r}$ is a scalar (and is therefore one-dimensional). In the feedback loop, the activation $\hat{r}$ is used to update the projection axis $\overrightarrow{w}$:

$$\overrightarrow{w_{n+1}} = \overrightarrow{w_n} + \eta\,\widehat{r_n}\,(\overrightarrow{a_n} - \hat{r}\,\overrightarrow{w_n})$$

The result of the update is that the projection axis $\overrightarrow{w}$ turns a small amount into the direction of the new input $\overrightarrow{a_n}$. The

degree to which this takes place depends on the distance of the point to the axis $\vec{a_n} - \hat{r}_n \vec{w_n}$, the activation $\hat{r}$ and the learning rate $\eta$, which is for example set to 0.001.

This iterative way of updating the projection axis perfectly fits with the iterative way of estimating gravity. Also, Oja's rule is a form of exponential smoothing, in which older values fade out exponentially.

In step 72, based on the one dimensional result $\hat{r}$ of the PCA of step 70, there is estimation of the inspiratory and expiratory directions.

For $\hat{r}$ it is desired that inspirations correspond to positive signal transitions and that expirations correspond to negative signal transitions, just as is the case in the reference signal.

Complying with this choice of directions lowers the estimation error. In addition, changes in the direction (flipping), cause extra transitions, which disturb the respiratory rate estimation.

Principal Component Analysis does not estimate the inspiratory direction; in this aspect it makes an arbitrary choice. For this reason, the estimation of the inspiratory direction is performed, using $\hat{r}$.

A feature that correlates strongly with the proper choice of the inspiratory direction is the skewness of $\hat{r}$. If $\hat{r}$ has the proper direction then the distribution of $\hat{r}$ is right-skewed (the skewness is positive). In this case, the samples in the left part of the distribution (the negative signal values) correspond to the inspiratory pause, in which the diaphragm and intercostal muscles are relaxed. The samples in the right part of the distribution (the positive signal values) correspond to the interval where the lungs are maximally filled.

Thus, a measure of skewness is used to determine the inspiratory direction from the different periods of time of the signal $\hat{r}$.

The signal at the output of step 70 is the same as the respiratory signal that comes out of block 72. Thus, step 72 is transparent to this signal. Step 72 estimates the direction and feeds this back to step 70, where corrections are made by flipping the direction of the projection axis.

Thus, when the direction of the projection axis is set correctly, inspirations correspond to upward transitions of this signal.

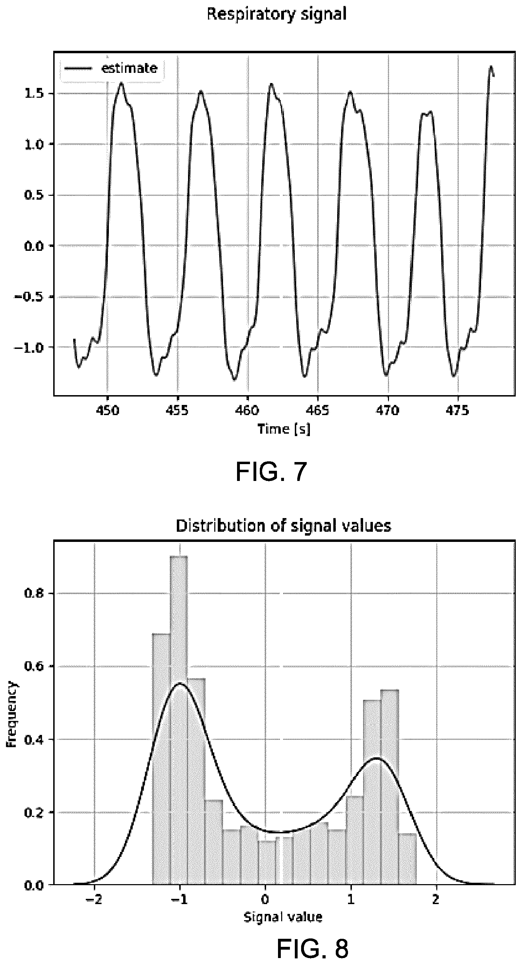

Figure 7 shows an estimate of the respiratory signal. Figure 8 shows the frequency distribution of the estimate. The skewness is 0.3, meaning the distribution is right-skewed.

The definition of skewness is as follows (with s the standard deviation and $\mu$ the mean):

$$g = \frac{\sum_{i=1}^{n}(\hat{r_i} - \mu)^3 / N}{s^3}$$

One approach is a recursive method. Instead of averaging, as in the definition above for skewness, exponential smoothing may be performed. Furthermore, $\hat{r}$ has a zero-mean and is normalized to have its standard deviation equal to one. Because of this, the skewness is approximated by the formula below.

An advantage of this approximation is that it fits very well in the recursive scheme of the other steps.

$$s_{n+1} = \alpha \hat{r}_n^3 + (1 - \alpha) s_n$$

The smoothing parameter $\alpha$ is for example equal to 0.0005. Using this in combination with a sampling rate of 16Hz gives a time constant of roughly 1 minute.

If the skewness $s_n$ is above a threshold (i.e. the distribution is sufficiently right-skewed) then the recursive process can continue without any changes; the projection axis $\vec{w}$ has the right direction and inspirations correspond to positive transitions. However, if the skewness $s_n$ goes below a threshold then the projection axis $\vec{w}$ has the wrong direction.

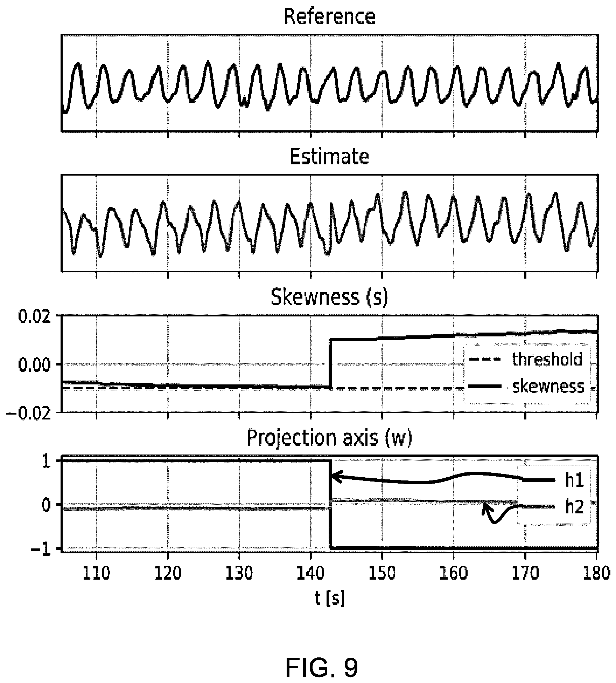

A correction must be made, as shown in Figure 9. Figure 9 shows an example of the correction of the inspiratory and expiratory directions. From top to bottom Figure 9 shows: the respiratory reference (from the RIP band), the respiratory estimate, the skewness with the threshold (dotted), and the projection axis (h1, h2).

Before t=143 s, the sign is inverted and therefore the skewness is decreasing. At t=143, the skewness drops below the threshold, causing the respiratory estimate, the skewness and the projection axis to be inverted.

The correction thus consists of inverting the sign of the projection axis $\vec{w}$, the skewness $s_n$ and the respiratory estimate $\hat{r}_n$ . The inversion of the signal of the projection axis $\vec{w}$ is a feedback to the PCA step, which is visible as a dashed arrow in Figure 6.

The inversion of the respiratory estimate $\hat{r}_n$ manifests itself as a discontinuity as shown in the second plot of Figure 9.

Setting the threshold to -0.01 prevents repeated inversion; when the skewness is decreasing, and $s_n$ goes below the threshold, then the sign of $s_n$ is is flipped, such that $s_n$ is above the threshold. Due to the flipping, a decreasing trend now becomes an increasing trend, and $s_n$ must change trend and decrease at least by twice the magnitude of the threshold before another inversion occurs Thus, a hysteresis effect is obtained, which prevents successive flipping.

The output of the unit 72 is the respiration signal with 16 Hz sampling rate, i.e. a reconstruction of a breathing signal corresponding to the reference signal shown in Figure 5.

The reference signal (from a RIP band) is a surrogate measurement for respiratory effort. By aiming to reconstruct this reference signal as closely as possible, by minimizing the difference between the respiratory signal from step 72 and the reference, the system generates a respiratory effort signal and can then be used to derive a respiration rate.

In this example, a respiration rate is estimated in step 74 by detecting peaks in a spectrogram, followed by statistical post-processing.

For this purpose, the respiration signal is down sampled to 4 Hz. This suffices for the purpose of respiration rate analysis. Then, a window is slid over the signal. The window length is for example 30 seconds (120 samples) and the time difference between successive windows may be 5 seconds.

Per window, a Fourier transform is applied (N=256, by zero-padding). The frequency resolution is:

$$\Delta f = \frac{f_s}{2\,N} 60 = \frac{240}{512} = 0.5\,\text{bpm}$$

For each window, the spectrum is normalized. The normalization constant is chosen such that the peak of a purely sinusoidal signal would have height 1.

The frequency $f_n$ of the highest peak in the spectrum of each window is selected and its height $h_n$ is recorded with it:

$$(f_n, h_n)$$

In the statistical post-processing, the peaks of 12 successive windows are combined into one estimate for the respiration rate, such that for each minute, a respiration rate estimate becomes available. First, peaks with a height above a threshold th are selected. Over these peaks, a weighted mean is taken in which the weights are the peak heights and the values are the respiratory rates of the peaks.

$$f_B = \frac{\sum_{n \text{ for which } h_n > th} h_n f_n}{\sum_{n \text{ for which } h_n > th} h_n}$$

If no peaks exist for which $h_n > th$ then for that minute no respiration rate is determined. The coverage is the number of intervals for which a respiration rate could be determined, divided by the total number of intervals.

The agreement between the estimated respiration rate and the reference has been analyzed and the results are shown in Figure 8.

The three plots in Figure 8 show the difference in respiration rate versus the mean respiration rate, comparing the respiratory rate estimated from the acceleration signal and that estimated from the reference signal. In particular, the horizontal axis in each case shows the mean of the two rates being compared and the vertical axis shows the difference between the two. The dashed lines are at -1.96 SD, 0 and +1.96 SD.

Each dot is a respiration rate over one minute. The dots are taken over all subjects, device positions, and postures and are grouped per posture (supine "sup", left and right). The diagrams are according to Bland-Altman.

The results show close agreement between the respiration rates calculated using the two approaches.

The example above is obtains both a respiratory signal and a respiration rate from the 3D acceleration signal. The processing to obtain the respiration rate is optional (step 74 in Figure 6), and there are other possible uses of the preceding respiratory signal, which may be considered to represent a 1D respiratory effort signal.

In another example, this 1D respiratory effort signal is used to detect sleep disordered breathing (SDB) events (hypopneas, and obstructive and central apneas).

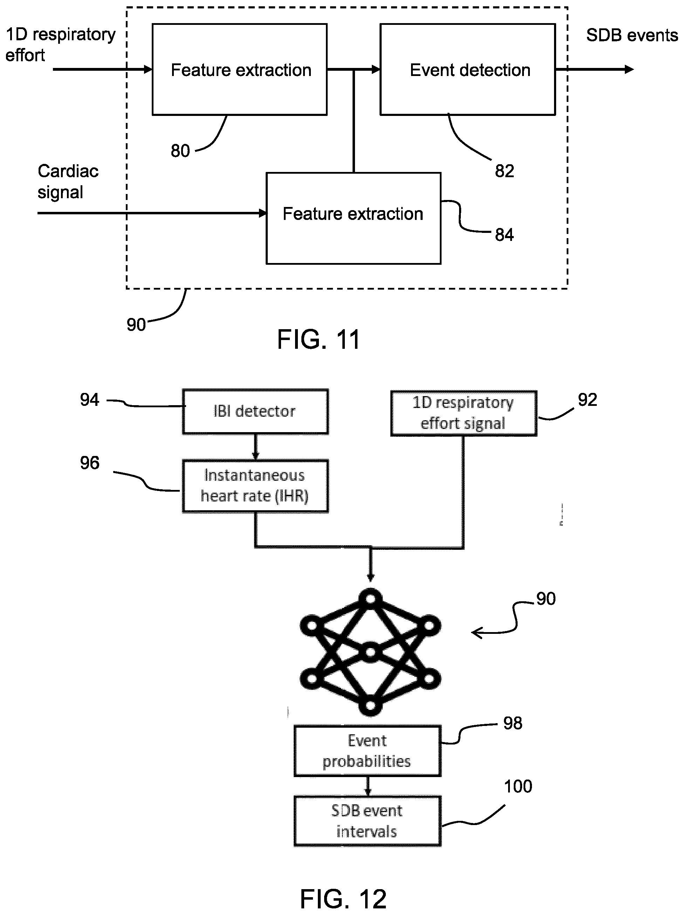

Figure 11 shows a system for detecting SDB events, from the 1D respiratory effort signal obtained at step 72 in Figure 6.

In step 80, meaningful features are extracted from the 1D respiratory signal. These features should express variations typically of is expected to be encountered from an effort signal in the presence (or vicinity) of an SDB event. These features can express characteristics of the amplitude of the respiratory signal, of its frequency, self-similarity of (repetitive) respiratory patterns, presence of artifacts (e.g. due to movements), entropy, etc.

Manually engineered features have been used for the purpose of SDB detection in (surrogate) respiratory signals obtained with different sensors, such as PPG as disclosed in the paper of G. B. Papini et. al. "Wearable monitoring of sleep-disordered breathing: estimation of the apnea-hypopnea index using wrist-worn reflective photoplethysmography," Sci Rep, vol. 10, no. 1, p. 13512, Dec. 2020.

The features can be extracted in windows of adequate size (e.g. 10-30 seconds, or longer, using sliding windows with overlap) an event detection algorithm 82 then detects DB events. For the event detection, a machine learning classifier may be used. The classifier can use any of a set of modern classification techniques, such as probabilistic (Bayesian) classifiers, support vector machines, logistic regression, or even neural networks trained with examples of respiratory features, and associated SDB events.

The output of the event detection algorithm can either be a flag indicating for a given period (or time) that an SDB event is present; alternatively, the algorithm can output a probability (or likelihood) of such an event.

Additionally, the classifier can output a flag or probability separately for each type of SDB event the classifier was trained with, for example separating obstructive apneas, central apneas, or hypopneas, or any combination of these. The classifier can also be used to detect the presence of other SDB events, such as respiratory-related arousals (RERAs).

Figure 11 additionally shows an optional extension by which a cardiac signal is processed by another feature extraction algorithm 84, so that respiratory and cardiac features are combined. The cardiac signal may be a sequence of inter-beat intervals (IBIs), or equivalent signals (e.g. instantaneous heart rate). In a preferred optional embodiment, this IBI signal can be derived from the same accelerometer signal used to obtain the 1D respiratory effort signal.

This can be achieved with a known technique for IBI detection in accelerometer signals, such as ballistocardiography or seismocardiography.

The cardiac feature extraction block 84 can be based on any combination of cardiac features known to be discriminative of SDB events, based e.g. on time and frequency analysis of IBI series, de-trended fluctuation analysis, multi-scale entropy, phase coordination, etc. Such manually engineered features have also been used for the purpose of SDB detection in IBI signals obtained with PPG in the Papini et. al. paper referenced above.

The use of manually engineered features is explained above. However, instead of (or in addition to) manually engineered features, the a deep neural network may be designed to learn discriminative respiratory (and optionally, cardiac) features from the input 1D respiratory effort signal (and optionally, cardiac signal).

For example the algorithms 80,82,84 may all be part of a neural network 90. When trained with adequate datasets with sufficient distinctive examples of input 1D respiratory effort signals, and corresponding annotations of SDB events, these neural networks, e.g. based on convolutional neural networks, can enhance the performance of the classifier by recognizing patterns in the input data, which cannot be easily discovered by humans when developing the manually engineered features.

Figure 12 shows a possible implementation of a system based on a neural network. Signals input to the neural network 90 are the 1D respiratory effort signal 92 and an instantaneous heart rate (IHR) signal 94 which has been derived from an inter-beat interval detector 96. This signal is for example interpolated at a fixed sampling rate.

The neural network 90 derives SDB event probabilities 98 for apnea and hypopnea events, and SDB event intervals 100 by applying thresholds to the event probabilities.

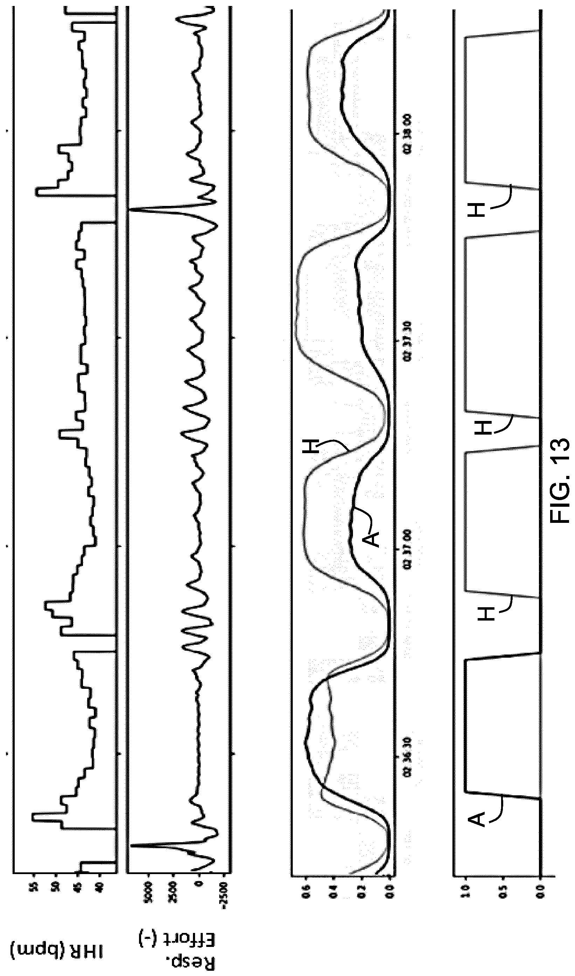

Figure 13 shows a set of possible signals. The top plot shows the IHR signal (over time), and the second plot shows the 1D respiratory effort signal (over time).

There are 4 SDB events, and the neural network is trained to distinguish between apneas A (central and obstructive) and hypopneas H.

The third plot shows output probabilities for apnea and hypopnea events as determined by the neural network, and the fourth plots shows that, after using predefined thresholds, the intervals corresponding to the duration of the events can be established.

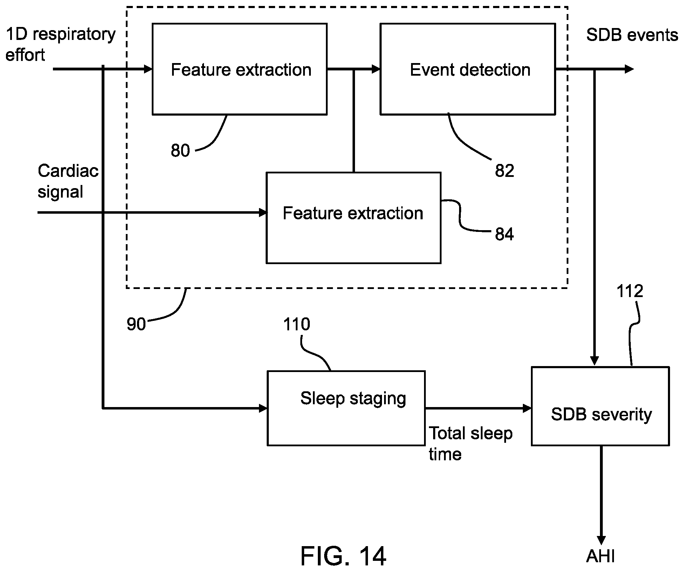

Figure 14 shows an extension to the example of Figure 11 in which the detected SDB events are also processed to estimate a level of SDB severity. For example, based on the detected apneas and hypopneas, the apnea-hypopnea index (AHI) can calculated.

In this system, the same 1D respiratory effort signal and cardiac signal are also as input to a sleep staging algorithm 110 which outputs as a minimum, the total sleep time. The sleep staging algorithm 110 may also provide more detailed sleep staging information, such as wake/light/deep/REM sleep, or even wake/N1/N2/N3/REM sleep. The total sleep time may be obtained by summing the time spent in any stage except wake.

By dividing the number of apnea and hypopnea events by the estimated total sleep time in a SDB severity algorithm 112, an estimate of the AHI may be obtained.

Respiratory effort related arousals (RERAs) may also be detected by the classifier. A respiratory disturbance index (RDI) may then also be derived, by dividing the number of RERAS, plus apneas and hypopneas, by the total sleep time.

Any alternative indication of SDB severity can also be computed by this approach, e.g. by integrating the SDB event probabilities, or by using any non-linear or weighted combination of detected events to privilege one type of SDB event over another, etc. In all of these cases sleep staging can be used to normalize the estimate of severity by the time spent asleep.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. (optional)

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to".

Any reference signs in the claims should not be construed as limiting the scope.

## Claims

1. A processor (60) for deriving a respiratory signal, comprising:
   an input (62) for receiving a 3-axis acceleration signal, wherein the processor is adapted to:

   (66) isolate a gravity vector from the 3-axis acceleration signal;
   (68) perform a coordinate transformation into a transformed 3-axis coordinate system in which a time-averaged gravity vector is aligned with a first axis of the transformed 3-axis coordinate system such that the remaining two axes of the transformed 3-axis coordinate system lie in a horizontal plane and comprise a projection of the 3-axis acceleration signal to the horizontal plane in the transformed 3-axis coordinate space; and
   (70) perform analysis of the components for the remaining two axes of the transformed 3-axis coordinate system, thereby to derive a 1 dimensional respiratory signal.

2. The processor of claim 1, further adapted to:
   (74) process the 1 dimensional respiratory signal to determine a respiration rate by using frequency analysis.

3. The processor of claim 2, adapted to determine the respiration rate from the 1 dimensional respiratory signal by applying a Fourier transform over a sliding time window and determining the frequency of the highest peak in the spectrum of each window, wherein the respiration rate is derived from the highest peaks for a set of successive windows

4. The processor of claim 1, further adapted to:
   (74) process the respiratory signal to determine sleep disordered breathing events from the 1 dimensional respiratory signal.

5. The processor of claim 4, adapted to determine sleep disordered breathing events by extracting features from the 1 dimensional respiratory signal.

6. The processor of claim 5, adapted to determine sleep disordered breathing events additionally based on analysis of a cardiac signal.

7. The processor of any one of claims 1 to 6, adapted to perform pre-processing (64) of the acceleration signal before isolating the gravity vector, wherein the pre-processing comprises sampling and low-pass filtering.

8. The processor of any one of claims 1 to 7, adapted to isolate the gravity vector from the 3-axis acceleration signal by implementing a recursive exponential smoothing function.

9. The processor of any one of claims 1 to 8, adapted to perform the coordinate transformation into the transformed 3-axis coordinate system by deriving a rotation matrix which maps the isolated gravity vector to the first axis.

10. The processor of any one of claims 1 to 8, adapted to perform the analysis to derive the 1 dimensional respiratory signal using principal component analysis or using machine learning.

11. The processor of any one of claims 1 to 10, adapted to estimate the inspiratory and expiratory directions from the 1 dimensional respiratory signal.

12. The processor of claim 11, adapted to estimate the inspiratory and expiratory directions by (72) determining a skewness value of the 1 dimensional respiratory signal, and optionally to perform an inversion if needed, such that one type of skewness corresponds to the inspiratory direction and another type of skewness corresponds to the expiratory direction.

13. A system for deriving a respiratory signal, comprising:

an accelerometer for generating a 3-axis acceleration signal; and
the processor of any one of claims 1 to 12 for processing the 3-axis acceleration signal.

14. A method for deriving a respiratory signal, comprising:

receiving a 3-axis acceleration signal;
isolating a gravity vector from the 3-axis acceleration signal;
performing a coordinate transformation into a transformed 3-axis coordinate system in which the isolated gravity vector is aligned with a first axis of the transformed 3-axis coordinate system, such that the remaining two axes of the transformed 3-axis coordinate system lie in a horizontal plane and comprise a projection of the 3-axis acceleration signal to the horizontal plane in the transformed 3-axis coordinate space; and
performing analysis of the components for the remaining two axes of the transformed 3-axis coordinate system, thereby to derive a 1 dimensional respiratory signal.

15. A computer program comprising computer program code which is adapted, when said computer program code is run on a computer, to implement the method of claim 14.


**Patentansprüche**

1. Prozessor (60) zum Ableiten eines Atemsignals, umfassend:
einen Eingang (62) zum Empfangen eines 3-Achsen-Beschleunigungssignals, wobei der Prozessor dazu angepasst ist:

(66) einen Schwerkraftvektor aus dem 3-Achsen-Beschleunigungssignal zu isolieren;
(68) eine Koordinatentransformation in ein transformiertes 3-Achsen-Koordinatensystem durchzuführen, in dem ein zeitgemittelter Schwerkraftvektor mit einer ersten Achse des transformierten 3-Achsen-Koordinatensystems ausgerichtet ist, sodass die verbleibenden zwei Achsen des transformierten 3-Achsen-Koordinatensystems in einer horizontalen Ebene liegen und eine Projektion des 3-Achsen-Beschleunigungssignals auf die horizontale Ebene im transformierten 3-Achsen-Koordinatenraum umfassen; und
(70) eine Analyse der Komponenten für die verbleibenden zwei Achsen des transformierten 3-Achsen-Koordinatensystems durchzuführen, um dadurch ein 1-dimensionales Atemsignal abzuleiten.

2. Prozessor nach Anspruch 1, der weiter dazu angepasst ist:
(74) das 1-dimensionale Atemsignal zu verarbeiten, um unter Verwendung von Frequenzanalyse eine Atemfrequenz zu bestimmen.

3. Prozessor nach Anspruch 2, der dazu angepasst ist, die Atemfrequenz durch Anwenden einer Fourier-Transformation über ein gleitendes Zeitfenster und Bestimmen der Frequenz der höchsten Spitze im Spektrum jedes Fensters aus dem 1-dimensionalen Atemsignal zu bestimmen, wobei die Atemfrequenz aus den höchsten Spitzen für einen Satz aufeinanderfolgender Fenster abgeleitet wird.

4. Prozessor nach Anspruch 1, der weiter dazu angepasst ist:
(74) das Atemsignal zu verarbeiten, um Ereignisse schlafbezogener Atmungsstörungen aus dem 1-dimensionalen Atemsignal zu bestimmen.

5. Prozessor nach Anspruch 4, der dazu angepasst ist, Ereignisse schlafbezogener Atmungsstörungen durch Extrahieren von Merkmalen aus dem 1-dimensionalen Atemsignal zu bestimmen.

**6.** Prozessor nach Anspruch 5, der dazu angepasst ist, Ereignisse schlafbezogener Atmungsstörungen zusätzlich basierend auf einer Analyse eines Herzsignals zu bestimmen.

**7.** Prozessor nach einem der Ansprüche 1 bis 6, der dazu angepasst ist, vor dem Isolieren des Schwerkraftvektors eine Vorverarbeitung (64) des Beschleunigungssignals durchzuführen, wobei die Vorverarbeitung ein Abtasten und ein Tiefpassfiltern umfasst.

**8.** Prozessor nach einem der Ansprüche 1 bis 7, der dazu angepasst ist, den Schwerkraftvektor durch Implementieren einer rekursiven exponentiellen Glättungsfunktion vom 3-Achsen-Beschleunigungssignal zu isolieren.

**9.** Prozessor nach einem der Ansprüche 1 bis 8, der dazu angepasst ist, die Koordinatentransformation in das transformierte 3-Achsen-Koordinatensystem durch Ableiten einer Rotationsmatrix durchzuführen, die den isolierten Schwerkraftvektor auf die erste Achse abbildet.

**10.** Prozessor nach einem der Ansprüche 1 bis 8, der dazu angepasst ist, die Analyse zum Ableiten des 1-dimensionalen Atemsignals unter Verwendung einer Hauptkomponentenanalyse oder unter Verwendung maschinellen Lernens durchzuführen.

**11.** Prozessor nach einem der Ansprüche 1 bis 10, der dazu angepasst ist, die Einatem- und die Ausatemrichtung aus dem 1-dimensionalen Atemsignal zu schätzen.

**12.** Prozessor nach Anspruch 11, der dazu angepasst ist, die Einatem- und die Ausatemrichtung durch Bestimmen (72) eines Schiefewerts des 1-dimensionalen Atemsignals zu schätzen und optional bei Bedarf eine Inversion durchzuführen, sodass ein Schiefetyp der Einatemrichtung und ein anderer Schiefetyp der Ausatemrichtung entspricht.

**13.** System zum Ableiten eines Atemsignals, umfassend:

einen Beschleunigungsmesser zum Erzeugen eines 3-Achsen-Beschleunigungssignals; und
den Prozessor nach einem der Ansprüche 1 bis 12 zum Verarbeiten des 3-Achsen-Beschleunigungssignals.

**14.** Verfahren zum Ableiten eines Atemsignals, umfassend:

Empfangen eines 3-Achsen-Beschleunigungssignals;
Isolieren eines Schwerkraftvektors aus dem 3-Achsen-Beschleunigungssignal;
Durchführen einer Koordinatentransformation in ein transformiertes 3-Achsen-Koordinatensystem, in dem der isolierte Schwerkraftvektor mit einer ersten Achse des transformierten 3-Achsen-Koordinatensystems ausgerichtet ist, sodass die verbleibenden zwei Achsen des transformierten 3-Achsen-Koordinatensystems in einer horizontalen Ebene liegen und eine Projektion des 3-Achsen-Beschleunigungssignals auf die horizontale Ebene im transformierten 3-Achsen-Koordinatenraum umfassen; und
Durchführen einer Analyse der Komponenten für die verbleibenden zwei Achsen des transformierten 3-Achsen-Koordinatensystems, um dadurch ein 1-dimensionales Atemsignal abzuleiten.

**15.** Computerprogramm, umfassend Computerprogrammcode, der dazu angepasst ist, wenn der Computerprogrammcode auf einem Computer ausgeführt wird, das Verfahren nach Anspruch 14 zu implementieren.

**Revendications**

**1.** Processeur (60) permettant de déduire un signal respiratoire, comprenant :
une entrée (62) pour recevoir un signal d'accélération à 3 axes, dans lequel le processeur est adapté pour :

(66) isoler un vecteur de gravité à partir du signal d'accélération sur 3 axes ;
(68) effectuer une transformation de coordonnées dans un système de coordonnées à 3 axes transformé dans lequel un vecteur de gravité moyenné dans le temps est aligné avec un premier axe du système de coordonnées à 3 axes transformé de telle sorte que les deux axes restants du système de coordonnées à 3 axes transformé se trouvent dans un plan horizontal, et comprennent une projection du signal d'accélération à 3 axes sur le plan horizontal dans l'espace de coordonnées à 3 axes transformé ; et
(70) effectuer une analyse des composantes pour les deux axes restants du système de coordonnées à 3 axes transformé, afin de déduire un signal respiratoire unidimensionnel.

2. Processeur selon la revendication 1, en outre adapté pour :
(74) traiter le signal respiratoire unidimensionnel pour déterminer un rythme respiratoire en utilisant une analyse de fréquence.

3. Processeur selon la revendication 2, adapté pour déterminer le rythme respiratoire à partir du signal respiratoire unidimensionnel en appliquant une transformée de Fourier sur une fenêtre temporelle glissante et en déterminant la fréquence du pic le plus élevé dans le spectre de chaque fenêtre, dans lequel le rythme respiratoire est déduit des pics les plus élevés pour un ensemble de fenêtres successives.

4. Processeur selon la revendication 1, en outre adapté pour :
(74) traiter le signal respiratoire pour déterminer des événements respiratoires perturbés du sommeil à partir du signal respiratoire unidimensionnel.

5. Processeur selon la revendication 4, adapté pour déterminer des événements respiratoires perturbés du sommeil en extrayant des caractéristiques à partir du signal respiratoire unidimensionnel.

6. Processeur selon la revendication 5, adapté pour déterminer des événements respiratoires perturbés du sommeil en se basant en outre sur une analyse d'un signal cardiaque.

7. Processeur selon l'une quelconque des revendications 1 à 6, adapté pour effectuer un prétraitement (64) du signal d'accélération avant d'isoler le vecteur de gravité, dans lequel le prétraitement comprend un échantillonnage et un filtrage passe-bas.

8. Processeur selon l'une quelconque des revendications 1 à 7, adapté pour isoler le vecteur de gravité à partir du signal d'accélération à 3 axes en mettant en œuvre une fonction de lissage exponentiel récursive.

9. Processeur selon l'une quelconque des revendications 1 à 8, adapté pour effectuer la transformation de coordonnées dans le système de coordonnées à 3 axes transformé en déduisant une matrice de rotation qui cartographie le vecteur de gravité isolé par rapport au premier axe.

10. Processeur selon l'une quelconque des revendications 1 à 8, adapté pour effectuer l'analyse afin de déduire le signal respiratoire unidimensionnel à l'aide d'une analyse de composantes principales ou à l'aide d'un apprentissage automatique.

11. Processeur selon l'une quelconque des revendications 1 à 10, adapté pour estimer les directions d'inspiration et d'expiration à partir du signal respiratoire unidimensionnel.

12. Processeur selon la revendication 11, adapté pour estimer les directions d'inspiration et d'expiration en (72) déterminant une valeur d'asymétrie du signal respiratoire unidimensionnel, et facultativement pour effectuer une inversion si nécessaire, de telle sorte qu'un type d'asymétrie corresponde à la direction d'inspiration et un autre type d'asymétrie corresponde à la direction d'expiration.

13. Système permettant de déduire un signal respiratoire, comprenant :

un accéléromètre pour générer un signal d'accélération à 3 axes ; et
le processeur selon l'une quelconque des revendications 1 à 12 pour traiter le signal d'accélération à 3 axes.

14. Procédé pour déduire un signal respiratoire, comprenant :

la réception d'un signal d'accélération à 3 axes ;
l'isolation d'un vecteur de gravité à partir du signal d'accélération sur 3 axes ;
la réalisation d'une transformation de coordonnées dans un système de coordonnées à 3 axes transformé dans lequel le vecteur de gravité isolé est aligné avec un premier axe du système de coordonnées à 3 axes transformé, de telle sorte que les deux axes restants du système de coordonnées à 3 axes transformé se trouvent dans un plan horizontal, et comprennent une projection du signal d'accélération à 3 axes sur le plan horizontal dans l'espace de coordonnées à 3 axes transformé ; et

la réalisation d'une analyse des composantes pour les deux axes restants du système de coordonnées à 3 axes transformé, afin de déduire un signal respiratoire unidimensionnel.

**15.** Programme informatique comprenant un code de programme informatique qui est adapté, lorsque ledit code de programme informatique est exécuté sur un ordinateur, pour mettre en œuvre le procédé de la revendication 14.

FIG. 1

FIG. 2

FIG. 3

FIG. 4

FIG. 5

3D acceleration

62

60

3 → Pre-processing 64

3

Gravity Removal 66

3

Horizontal Projection 68

2

PCA 70

sign

1

IE Direction Estimation 72

1 → Respiratory signal

Respiration Rate Estimation 1 → Respiration rate

74

FIG. 6

FIG. 7

FIG. 8

FIG. 9

FIG. 10

FIG. 11

FIG. 12

FIG. 13

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015018752 A
- EP 2263532 A
- US 2009062628 A1

**Non-patent literature cited in the description**

- **J. KARHUNEN** ; **J. JOUTSENSALO**. Generalizations of Principal Component Analysis, Optimization Problems, and Neural Networks. *Neural Networks*, 1995, vol. 8 (4), 549-562
- **G. B. PAPINI**. Wearable monitoring of sleep-disordered breathing: estimation of the apnea-hypopnea index using wrist-worn reflective photoplethysmography. *Sci Rep*, December 2020, vol. 10 (1), 13512